Europäisches Patentamt

European Patent Office

Office européen des brevets

(11) Veröffentlichungsnummer: **0 291 944 B1**

(12) **EUROPÄISCHE PATENTSCHRIFT**

(45) Veröffentlichungstag der Patentschrift: 25.09.91

(51) Int. Cl.⁵: **A61K 6/04**, C22C 1/04, B22F 1/00

(21) Anmeldenummer: **88107926.3**

(22) Anmeldetag: **18.05.88**

(54) **Verfahren zur Herstellung von Legierungspulvern für Dentalamalgame.**

(30) Priorität: **21.05.87 DE 3717048**

(43) Veröffentlichungstag der Anmeldung:
**23.11.88 Patentblatt 88/47**

(45) Bekanntmachung des Hinweises auf die
Patenterteilung:
**25.09.91 Patentblatt 91/39**

(84) Benannte Vertragsstaaten:
**AT BE CH DE ES FR GB GR IT LI LU NL SE**

(56) Entgegenhaltungen:
**DE-C- 3 240 256
US-A- 4 235 631**

**CHEMICAL ABSTRACTS, Band 94, Nr. 6, Februar 1981, Columbus, OH (US); Seite 377, Nr. 36380d**

(73) Patentinhaber: **Degussa Aktiengesellschaft
Weissfrauenstrasse 9
W-6000 Frankfurt am Main 1(DE)**

(72) Erfinder: **Groll, Werner, Dipl.Ing.Dr.
Gartenstr. 5
W-8755 Alzenau-Hörstein(DE)**
Erfinder: **Hathaway, Doris
Lahnstr. 20
W-6450 Hanau 7(DE)**
Erfinder: **Schöck, Gernot, Dipl.-Ing.
Bahnhofstr. 56
W-6454 Bruchköbel(DE)**

**Beschreibung**

Die Erfindung betrifft ein Verfahren zur Herstellung von Legierungspulvern für Dentalamalgame aus Silber, Kupfer, Zinn und gegebenenfalls Zusätzen von Indium, Zink, Palladium, Keramik- und/oder Glaspulver durch mechanisches Zerkleinern eines gepressten und gesinterten Formkörpers. Diese Pulver werden mit Quecksilber trituriert und finden als Amalgame zur Füllung von Zahnkavitäten Verwendung.

Silberamalgame haben sich über Jahrzehnte als Füllungsmaterial für durch Zahnerkrankungen geschädigte Zähne bewährt, was insbesondere auf ihre hohe mechanische Festigkeit, ihre Langlebigkeit und ihre unproblematische Applikation zurückzuführen ist. Die Herstellung des Amalgams erfolgt beim Zahnarzt aus einem geeigneten Legierungspulver und reinem Quecksilber.

Die Legierungspulver bestehen normalerweise aus den Elementen Silber, Kupfer und Zinn, häufig auch aus etwas Zink, wobei die Silber-Gehalte von 24 bis 93 Gew.% schwanken (z.B. DE-OS 25 11 194, US-PSen 40 39 329, 39 75 192, 40 30 918, 37 62 917, 38 71 876). Teilweise werden auch andere Zusätze, wie Indium (z.B. US-PS 40 30 918) oder Palladium (US-PS 43 74 085) verwendet.

Die Herstellung der Legierungspulver erfolgt gewöhnlich schmelzmetallurgisch, entweder durch Verdüsung der Schmelze oder durch spanabhebende Zerkleinerung eines gegossenen Formkörpers (z.B. Barren oder Bolzen).

Beim Verdüsen wird das Pulver direkt aus der Schmelze hergestellt, indem der Schmelzstrahl über einen Trichter durch eine mit Wasser oder Gas beaufschlagte Ringdüse geleitet und zerstäubt wird. In den überwiegend sphärisch geformten Pulverpartikeln liegen die Legierungphasen, z.B. $Ag_3Sn$ und $Cu_3Sn$, aufgrund der hohen Abkühlgeschwindigkeit bei diesem Prozeß fein verteilt vor. Mit solchen Pulvern werden -insbesondere bei geringen Silbergehalten - Amalgame mit hoher Festigkeit erzielt. Wegen der Kugelform der Pulverpartikel und der damit verbundenen hohen Schüttdichte ist der Quecksilberbedarf zur Herstellung eines pastösen Stopfkörpers zwar geringer als bei Spanamalgamen, doch sind die Verarbeitungseigenschaften nicht optimal, da die einzelnen Partikel beim Stopfen zu leicht aneinander vorbeigleiten und so dem Stopfdruck ausweichen. Die Verarbeitungseigenschaften von solchen sog. "Kugelamalgamen" reagieren außerdem deutlich empfindlicher auf Dosierungsungenauigkeiten des Quecksilbers als Spanamalgame. Die hohen Fertigungskosten der verdüsten Legierung, die zu einem großen Teil durch die geringe Ausbeute an verwertbarem Pulver entstehen, sind ebenfalls nachteilig.

Beim Zerspanen von gegossenen Formkörpern kommt es wegen der relativ geringen Abkühlgeschwindigkeit bei der Herstellung des zu fräsenden Gußbarrens zu Steigerungen und zu einer groben Verteilung der Phasen $Ag_3Sn$ und $Cu_3Sn$ im Formkörper und im Legierungspulver.

Dies wirkt sich negativ auf die Abbindereaktion mit dem Quecksilber aus. Auch die technischen Eigenschaften, wie z.B. Abbindeexpansion und Festigkeit werden negativ beeinflußt. Zudem ist die Ausbeute an verwertbarem Pulver mit 30 - 50 % sehr klein, so daß durch nachgeschaltete Mahl prozesse und evt. entstehende Scheideabfälle erhöhte Kosten anfallen. Ein Vorteil der Spanamalgame gegenüber den Kugelamalgamen ist ihre sehr gute Verarbeitbarkeit.

In der DE-PS 32 40 256 wird ein sintertechnologisches Verfahren zur Herstellung eines Amalgampulvers beschrieben. Zunächst wird ein verdüstes Legierungspulver aus Silber-Zinn-Kupfer hergestellt, das nachfolgend kompaktiert und gesintert wird. Der poröse, gesinterte Legierungsformkörper wir dann zerspant. Auf diesem Weg wird eine Qualitätsverbesserung des Amalgams erreicht. Der Nachteil dieses Verfahrens liegt in den hohen Produktionskosten, die hauptsächlich durch die Herstellung des Legierungspulvers über das Verdüsen entstehen.

Es war daher Aufgabe der vorliegenden Erfindung, ein Verfahren zur Herstellung von Legierungspulvern für Dentalamalgame aus Silber, Kupfer, Zinn und gegebenenfalls Zusätzen von Indium, Zink, Palladium, Keramik- und/oder Glaspulver durch mechanisches Zerkleinern eines gepressten und gesinterten Formkörpers zu entwickeln, das eine sehr feine Mikrostruktur des Legierungspulvers gewährleistet und mit hohen Ausbeuten an verwertbarem Amalgampulver durchgeführt werden kann und damit kostengünstig ist.

Diese Aufgabe wird erfindungsgemäß dadurch gelöst, daß der Formkörper durch Mischen und Pressen der elementaren Metallpulver Silber, Kupfer, Zinn und gegebenenfalls der Zusätze erzeugt wird und das Sintern zwischen 150°C und der Solidustemperatur der entstehenden Legierung solange durchgeführt wird, bis eine homogene Verteilung des Zinns in den Silber- und Kupferpartikeln erreicht ist.

Die einzelnen Pulver werden intensiv gemischt, so daß eine agglomeratfreie Mischung der Pulver entsteht. Dieser Pulvermischung wird in einer geeigneten Form und Presse zu einem Grünling verdichtet und anschließend gesintert. Danach wird der gesinterte Körper (Barren, Bolzen o.ä.) beispielsweise in einer Fräsmaschine zerspant und die benötigte Fraktion abgetrennt.

Vorzugsweise erfolgt der Sinterprozess in zwei Stufen, wobei die erste Stufe während 1 bis 20 Stunden unterhalb des Schmelzpunktes des Zinn (232°C) und die zweite Stufe bei Temperaturen zwischen 232°C

EP 0 291 944 B1

und der Solidustemperatur der entstehenden Legierung solange durchgeführt wird, bis eine homogene Verteilung des Zinns in den Silber- und Kupferpartikeln erreicht ist.

Als besonders geeignet haben sich Metallpulver mit einer Teilchengröße kleiner als 70 μm erwiesen, da diese zum einen eine hinreichend feine Phasenverteilung gewährleisten und zum anderen eine relativ kurze Sinterdauer ermöglichen, in der das gesamte Zinn in die Kupfer- bzw. Silberpartikeln diffundieren kann.

Eine noch feinere Verteilung der einzelnen silber- bzw. kupferreichen Phasen wird erreicht, wenn die Pulvermischung vor dem Pressen einem Mahlprozeß (z.B. in einem schnellaufenden Attritor) unterworfen wird. Die Phasengröße der silber- bzw. kupferreichen Partikel (vorwiegend $Ag_3Sn$ und $Cu_3Sn$), die so erreichbar ist, liegt im Bereich von wenigen Mikrometern und ist somit vergleichbar mit der von verdüsten Pulvern.

Aufgrund der intensiven Verarbeitung des Pulvers während des Mahlprozesses besteht an Luft die Gefahr einer Erhöhung der Sauerstoffkonzentration im Legierungspulver. Dies wirkt sich negativ auf die Amalgameigenschaften aus. Der Mahlprozeß wird deshalb bevorzugt unter Schutzgasbedingungen durchgeführt.

Außerdem ist es vorteilhaft, den zu zerspannenden Formkörper durch isostatisches Pressen herzustellen.

Die Sintertemperaturen sind entsprechend der jeweiligen Pulver- bzw. Legierungszusammensetzung zu wählen. Der Sinterprozeß wird vorzugsweise zweistufig durchgeführt, wobei der gepreßte Grünling zunächst unterhalb der Schmelztemperatur von Sn (232°C) 1 - 20 Stunden gesintert wird, so daß sämtliches elementares Zinn in die Silber-Kupferpartikel diffundieren kann. Im weiteren Verlauf wird die Sintertemperatur auf Werte zwischen dem Schmelzpunkt von Zinn und der Solidustemperatur der Legierung angehoben und dort solange gehalten, bis eine homogene Verteilung des Zinns in dem Kupfer- bzw. Silberpartikeln erreicht ist. Da weder beim einstufigen noch beim zweistufigen Sintern die Silber- und Kupferpartikel aufschmelzen, bleibt deren ursprüngliche Korngröße im wesentlichen erhalten. Eine kleine Korngröße läßt sich bei der Herstellung von elementaren Metallpulvern leichter einstellen als bei der Herstellung von Legierungspulvern.

Die technischen Eigenschaften der mit den erfindungsgemäß hergestellten Legierungspulvern erzeugten Amalgamen erreichen bzw. übertreffen die Anforderungen der ADA-bzw. ISO-Spezifikationen:

| | | |
|---|---|---|
| ADA: | Dimensionsänderung währen des Abbindens: | + 2μm/cm |
| | Creep            3,0 % | |
| | Druckfestigkeit nach einer Stunde            80 MPa | |
| ISO: | Dimensionsänderung während des Abbindens: | -10 bis 20 μm/cm |
| | Creep            3,0 % | |
| | Druckfestigkeit nach einer Stunde            50 MPa | |
| | Druckfestigkeit nach 24 Stunden            300 MPa | |

Die Amalgame besitzen die den Spanamalgamen eigene, gute Verarbeitbarkeit. Insbesondere im Bereich niedriger Ag-Gehalte werden mit dem erfindungsgemäß hergestellten Legierungspulvern z.T. deutlich bessere Amalgameigenschaften erreicht als mit einem Legierungspulver der gleichen Zusammensetzung, das auf gußtechnischem Wege hergestellt wurde.

Die Ausbeute an gefrästem Pulver, das die für die Amalgamherstellung geeignete Partikelgröße aufweist, ist im Vergleich zu gußtechnischen hergestellten Amalgampulvern mit 70 - 85 % nahezu doppelt so hoch, so daß ein aufwendiges, die Eigenschaften beeinflussendes Mahlen des gröberen, gefrästen Pulvers auf sehr geringe Mengen beschränkt bleibt.

Folgende Beispiele sollen das erfindungsgemäße Verfahren näher erläutern:

1. 50 % Silberpulver (kleiner 63 μm), 30 % Zinnpulver (Fa. Merck, Artn.Nr. 7807) und 20 % Kupferpulver (kleiner 63 μm) wurden in einem Mischer ca. 15 min. gemischt, anschließend in eine dicht verschließbare Gummiform (Zylinder) abgefüllt und bei 1000 MPa isostatisch zu einem Bolzen gepreßt. Der Preßling wurde acht Stunden bei 300°C unter Argon-Atmosphäre gesintert. Entsprechend den Ergebnissen der Gefüge- und Mikrosondenuntersuchungen war nach dem Sintern keines der elementaren Pulver mehr vorhanden. Die intermetallsichen Phasen $Ag_3Sn$ und $Cu_3Sn$ waren entstanden, indem Zinn in das Silber bzw. Kupfer diffundiert war. Nach dem Zerspanen in einer Feilmaschine wurde die Pulverfraktion kleiner 63 μm abgesiebt und vier Stunden unter Formiergas ($N_2/H_2$ = 80/20) bei 180°C geglüht. Das Triturieren mit Quecksilber erfolgte in einem Duomat[R] (Fa. Degussa) mit einem Anmischverhältnis Legierungspulver zu Quecksilber von 1 : 1,2. Die Mischzeit betrug 40 Sekunden. Die Daten des mit diesem Legierungspulver hergestellten Amalgams sind in der Tabelle aufgeführt.

2. 70 % Silberpulver (kleiner 63 μm), 27 % Zinnpulver (Fa. Merck, Art.Nr. 7807) und 3 % Kupferpulver (kleiner 63 μm) wurden in einem Mischer ca. 15 min. gemischt, anschließend in eine dicht verschließbare Gummiform (Zylinder) abgefüllt und bei 1800 MPa isostatisch zu einem Bolzen gepreßt. Der Preßling wurde sechs Stunden bei 300° C unter Argon-Atmosphäre gesintert. Entsprechend den Ergebnissen der Gefüge- und Mikrosondenuntersuchungen war nach dem Sintern keines der elementaren Pulver mehr vorhanden. Nach dem Zerspanen in einer Feilmaschine wurde die Pulverfraktion kleiner 63 µm abgesiebt und vier Stunden unter Fomiergas ($N_2/H_2$) = 80/20) bei 240° C geglüht. Das Triturieren mit Quecksilber erfolgte in einem Duomat$^R$ (Fa. Degussa) mit einem Anmischverhältnis Legierungspulver zu Quecksilber 1 : 1,2. Die Daten des mit diesem Legierungspulver hergestellten Amalgams sind der Tabelle zu entnehmen.

3. Silber-, Kupfer und Zinnpulver mit der dem Beispiel 2 entsprechenden Zusammensetzung wurden ebenso gemischt und gepreßt (800 MPa). Der Sinterprozeß wurde zweistufig durchgeführt, wobei zuerst 6 Stunden bei 230° C und anschließend 2 Stunden bei 400° C unter Argon- Atmosphäre gesintert wurde. Die weiteren Herstellungsschritte wurden wie in Beispiel 2 beschrieben gewählt. Die Daten des Amalgams sind der Tabelle zu entnehmen.

4. 45 % Ag-Pulver (kleiner 20 µm), 24 % Kupferpulver (kleiner 20 µm) und 31 % Zinnpulver (Fa. Merck, Art-Nr. 7807) wurden entsprechend Beispiel 1 gemischt und gepreßt (Preßdruck 120 MPa). Das Sintern erfolgte wie in Beispiel 3 zweistufig bei den gleichen Temperaturen, jedoch unter Formiergas ($N_2/H_2$ = 80/20). Die Gefügeuntersuchungen im Lichtmikroskop und an der Mikrosonde ergaben, daß die Legierung aus einer Kupfer-Zinn- und einer Silber-Zinn-Phase besteht, die mittels Röntgenfeinstrukturuntersuchung als $Ag_3Sn$ und $Cu_3Sn$ identifiziert wurden. Die reinen Ausgangspulver treten im Gefüge nicht mehr auf. Die gesinterte Legierung wurde mit einem Walzenfräser gefräst. Die Ausbeute an der für die Amalgamherstellung verwertbaren Pulverfraktion kleiner 63 µm betrug 82 %. Die Wärmebehandlung der ausgesiebten Fraktion kleiner 63 µm erfolgte vier Stunden bei 80° C unter Formiergas ($N_2/H_2$ = 80/20). Die Daten des mit diesem Legierungspulver hergestellten Amalgams sind in der Tabelle aufgeführt. Das Amalgam ist $\gamma_2$-frei.

5. Den Silber-, Kupfer- und Zinn-Pulvern entsprechend der Zusammensetzung in Beispiel 4 wurden 5 Vol.% Glaspulver ($\leq$ 5 µm) zugegeben. Mischen, Pressen, Sintern und Fräsen erfolgten analog Beispiel 4. Die Glaspartikel lagen nach dem Sintern feinverteilt in der Matrix aus $Cu_3Sn$ und $Ag_3Sn$ vor. Die Ausbeute beim Fräsen betrug 76 %. Nach der Wärmebehandlung der Pulverfraktion kleiner 63 µm (wie Beispiel 4) wurde das Legierungspulver mit Quecksilber trituriert. Die Eigenschaften dieses Amalgams sind ebenfalls in der Tabelle aufgeführt.

6. Eine Pulvermischung entsprechend Beispiel 4 (150 g) wurde in einem Attritor, dessen Mahlgefäß aus Zirkonoxid besteht und das wassergekühlt ist, mit Zirkonoxidkugeln (600 g) in Alkohol trituriert. Die Umdrehungszahl betrug 1200 U/min., die Mahldauer, 4 Stunden. Nach Abtrennung des Mahlmediums wurde das gemahlende Pulver in eine Gummiform gefüllt und analog Beispiel 4 weiterverarbeitet. Nach dem Sintern lag ein Gefüge vor, in dem die $Ag_3Sn$ und $Cu_3Sn$-Phasen äußerst feinverteilt vorzufinden waren. Die Phasengröße lag im Bereich von wenigen µm.

EP 0 291 944 B1

Tabelle

| Beispiel | Mischungs- verhältnis Leg. : Hg | Anmisch- zeit in s | Creep in % | Längenänd. während d. Erhärtens in um/cm | Druckfestigkeit in MPa nach | | |
|---|---|---|---|---|---|---|---|
| | | | | | 1 h | 24 h | 7 d |
| Beispiel 1 | 1 : 1,2 | 40 | 0,45 | + 10 | 90 | 340 | 370 |
| Beispiel 2 | 1 : 1,2 | 30 | 1,6 | - 11 | 85 | 290 | 310 |
| Beispiel 3 | 1 : 1,2 | 30 | 1,2 | - 8 | 130 | 330 | 385 |
| Beispiel 4 | 1 : 1,3 | 30 | 0,22 | + 4 | 126 | 385 | 450 |
| Beispiel 5 | 1 : 1,3 | 30 | 0,25 | + 5 | 105 | 330 | 360 |

**Patentansprüche**

1. Verfahren zur Herstellung von Legierungspulvern für Dentalamalgame aus Silber, Kupfer, Zinn und gegebenenfalls Zusätzen von Indium, Zink, Palladium, Keramik- und/oder Glaspulver durch mechanisches Zerkleinern eines gepressten gesinterten Formkörpers,

dadurch gekennzeichnet,
daß der Formkörper durch Mischen und Pressen der elementaren Metallpulver Silber, Kupfer, Zinn und gegebenenfalls der Zusätze erzeugt wird und das Sintern zwischen 150°C und der Solidustemperatur der entstehenden Legierung solange durchgeführt wird, bis eine homogene Verteilung des Zinns in den Silber- und Kupferpartikeln erreicht ist.

2. Verfahren nach Anspruch 1,
dadurch gekennzeichnet,
daß der Sinterprozess in zwei Stufen erfolgt, wobei die erste Stufe während 1 bis 20 Stunden unterhalb des Schmelzpunktes des Zinns (232°C) und die zweite Stufe bei Temperaturen zwischen 232°C und der Solidustemperatur der entstehenden Legierung solange durchgeführt wird, bis eine homogene Verteilung des Zinn in den Silber-und Kupferpartikeln erreicht ist.

3. Verfahren nach Anspruch 1 und 2,
dadurch gekennzeichnet,
daß die Teilchengröße der eingesetzten Pulver weniger als 70 μm beträgt.

4. Verfahren nach Anspruch 1 bis 3,
dadurch gekennzeichnet,
daß die Pulver vor dem Pressen einem Mahlprozess unterworfen werden.

5. Verfahren nach Anspruch 1 bis 4,
dadurch gekennzeichnet,
daß der Mahlprozess unter Schutzgas durchgeführt wird.

6. Verfahren nach Anspruch 1 bis 5,
dadurch gekennzeichnet,
daß der Formkörper durch isostatisches Pressen hergestellt wird.

**Claims**

1. A process for the production of alloy powders for dental amalgams from silver, copper, tin and, optionally, additions of indium, zinc, palladium, ceramic and/or glass powder by mechanical size-reduction of a sintered compact, characterized in that the compact is produced by mixing and compressing the elemental metal powders silver, copper, tin and, optionally, the additions and sintering is carried out between 150°C and the solidus temperature of the alloy formed until the tin is homogeneously dispersed in the silver and copper particles.

2. A process as claimed in claim 1, characterized in that the sintering process is carried out in two steps, the first step being carried out for 1 to 20 hours at a temperature below the melting point of tin (232°C) and the second step being carried out at temperatures between 232°C and the solidus temperature of the alloy to be formed until the tin is homogeneously dispersed in the silver and copper particles.

3. A process as claimed in claims 1 and 2, characterized in that the particle size of the powders used is less than 70 μm.

4. A process as claimed in claims 1 to 3, characterized in that the powders are ground before being compressed.

5. A process as claimed in claims 1 to 4, characterized in that the grinding process is carried out in an inert gas atmosphere.

6. A process as claimed in claims 1 to 5, characterized in that the compact is produced by isostatic compression.

**Revendications**

1. Procédé pour la préparation de poudres d'alliages pour amalgames dentaires à partir d'argent, cuivre,

EP 0 291 944 B1

étain et éventuellement d'additions d'indium, zinc, palladium, de poudre de céramique et/ou de poudre de verre, par fragmentation mécanique d'un corps moulé comprimé et fritté, caractérisé en ce que le corps moulé est obtenu par mélange et compression de poudres métalliques élémentaires d'argent, cuivre, étain et éventuellement des produits d'addition, et qu'on effectue le frittage entre 150°C et la température de solidification de l'alliage résultant, jusqu'à l'obtention d'une répartition homogène de l'étain dans les particules d'argent et de cuivre.

2. Procédé selon la revendication 1, caractérisé en ce que le processus de frittage est effectué en deux étapes, la première étape étant effectuée pendant 1 à 20 heures au-dessous du point de fusion de l'étain (232°C) et la seconde étape étant effectuée à des températures comprises entre 232°C et la température de solidification de l'alliage résultant, jusqu'à l'obtention d'une répartition homogène de l'étain dans les particules d'argent et de cuivre.

3. Procédé selon la revendication 1 ou 2, caractérisé en ce que la taille de particules de la poudre utilisée est inférieure à 70 $\mu$m.

4. Procédé selon l'une quelconque des revendications 1 à 3, caractérisé en ce que les poudres sont soumises à un processus de broyage avant la compression.

5. Procédé selon l'une quelconque des revendications 1 à 4, caractérisé en ce que le processus de broyage est effectué sous gaz protecteur.

6. Procédé selon l'une quelconque des revendications 1 à 5, caractérisé en ce que le corps moulé est préparé par compression isostatique.

7